# EUROPEAN PATENT APPLICATION

(11) **EP 1 186 617 A1**
(43) Date of publication of application: **13.03.2002**
(21) Application number: 01921872.6
(22) Date of filing: 18.04.2001
(51) Int. Cl.: C07K 16/24, C12N 15/13, C12P 21/08, A61K 39/395

(54) **NOVEL RECOMBINANT ANTIBODY, AMINO ACID SEQUENCES OF CDRS THEREOF AND GENES ENCODING THE SAME**

(30) Priority: 19.04.2000 JP 2000117394
(71) Applicant: SUNTORY LIMITED, Osaka-shi, Osaka 530-8203 (JP)
(72) Inventor: FUKUDA, Yoshiaki, Ibaraki-shi, Osaka 567-0827 (JP); NAGAHIRA, Kazuhiro, Nagaokakyo-shi, Kyoto 617-0817 (JP); NAKANISHI, Toshihiro, Ibaraki-shi, Osaka 567-0824 (JP)
(74) Representative: HOFFMANN - EITLE
(86) International application number: JP0103308
(87) International publication number: WO0179298

(57) **Abstract**

There are provided an H chain polypeptide of a recombinant antibody against human TNFα or its fragment, having at least one of the following amino acid sequences:
a) as CDR-H1, Gly-Tyr-Thr-Phe-Thr-Asn-Tyr-Gly-Met-Asn;
2) as CDR-H2, 
Trp-Ile-Asn-Thr-Tyr-Thr-Gly-Glu-Pro-Thr-Tyr-Ala-
Asp-Asp-Phe-Lys-Gly;
 and
c) as CDR-H3, Tyr-Asp-Tyr-Asp-Gly-Phe-Asp-Tyr, an L chain polypeptide of a recombinant antibody against human TNFα having at least one of the following amino acid sequences:
   a') as CDR-L1. Thr-Ala-Ser-Ser-Ser-Val-Ser-Phe-Ser-Tyr-Leu-His;
   b') as CDR-L2, Tyr-Ser-Thr-Ser-Asn-Leu-Ala-Ser; and
   c') as CDR-L3, His-Gln-Tyr-Leu-Arg-Ser-Pro-Tyr-Thr,
and a humanized antibody against human TNFα comprising the above-described H chain polypeptide or its fragment and the L chain polypeptide, or its fragment. There is further provided, a method for producing a humanized anti-TNFα antibody which comprises transforming host cells by an expression vector having a gene encoding the above-described antibody, etc. and culturing the cells.

SEQUENCE LISTING
<110> Suntory Limited
<120> Novel recombinant antibody, amino acid sequences of its
complementarity determining regions and genes encoding the same
<130> YCT-588
<160> 19

<210> SEQ ID NO:1
<211> 10
<212> PRT
<213> mouse
<220>
<223> CDR-H1 of anti-human TNF-alpha antibody
<400> 1
Gly Tyr Thr Phe Thr Asn Tyr Gly Met Asn
                5                   10

<210> SEQ ID NO:2
<211> 17
<212> PRT
<213> mouse
<220>
<223> CDR-H2 of anti-human TNF-alpha antibody
<400> 2
Trp Ile Asn Thr Tyr Thr Gly Glu Pro Thr Tyr Ala Asp Asp Phe
                5                   10                  15
Lys Gly

<210> SEQ ID NO:3
<211> 8
<212> PRT
<213> mouse
<220>
<223> CDR-H3 of anti-human TNF-alpha antibody
<400> 3
Tyr Asp Tyr Asp Gly Phe Asp Tyr
                5

<210> SEQ ID NO:4
<211> 12
<212> PRT
<213> mouse
<220>
<223> CDR-L1 of anti-human TNF-alpha antibody
<400> 4
Thr Ala Ser Ser Ser Val Ser Phe Ser Tyr Leu His
                5                   10

<210> SEQ ID NO:5
<211> 8
<212> PRT
<213> mouse
<220>
<223> CDR-L2 of anti-human TNF-alpha antibody
<400> 5
Tyr Ser Thr Ser Asn Leu Ala Ser

                5

<210> SEQ ID NO:6
<211> 9
<212> PRT
<213> mouse
<220>
<223> CDR-L3 of anti-human TNF-alpha antibody
<400> 6
His Gln Tyr Leu Arg Ser Pro Tyr Thr
                5

<210> SEQ ID NO:7
<211> 351
<212> DNA
<213> mouse
<220>
<223> H-chain CDR region of anti-human TNF-alpha antibody
<400> 7
cag gtg aag ctg ctc gag tct ggg gga ggc gtg gtc cag cct ggg agg 48
Gln Val Lys Leu Leu Glu Ser Gly Gly Gly Val Val Gln Pro Gly Arg
1               5                   10                  15
tcc ctg aga ctc tcc tgt gca gcc tct gga tac acc ttt act aac tat 96
Ser Leu Arg Leu Ser Cys Ala Ala Ser Gly Tyr Thr Phe Thr Asn Tyr
            20                  25                  30
ggt atg aac tgg gtc cgc cag gct cca ggc aag ggg ttg aag tgg gtg 144
Gly Met Asn Trp Val Arg Gln Ala Pro Gly Lys Gly Leu Lys Trp Val
        35                  40                  45

gca tgg ata aac act tat aca ggt gag cca acc tac gca gac gac ttc 192
Ala Trp Ile Asn Thr Tyr Thr Gly Glu Pro Thr Tyr Ala Asp Asp Phe
    50                  55                  60
aag ggc cga ttc acc att tcc tta gac aat tcc aag aac aca gcg tat 240
Lys Gly Arg Phe Thr Ile Ser Leu Asp Asn Ser Lys Asn Thr Ala Tyr
65                  70                  75                  80
ctg gaa gtg aag agc ctg caa act gag gac acg ggt gtc tat tac tgt 288
Leu Glu Val Lys Ser Leu Gln Thr Glu Asp Thr Gly Val Tyr Tyr Cys
                85                  90                  95
gca aga tat gat tat gac gga ttt gac tac tgg ggc cag gga acc ctg 336
Ala Arg Tyr Asp Tyr Asp Gly Phe Asp Tyr Trp Gly Gln Gly Thr Leu
            100                 105                 110
gtc acc gtc tcc tca 351
Val Thr Val Ser Ser
        115

<210> SEQ ID NO:8
<211> 324
<212> DNA
<213> mouse
<220>
<223> L-chain CDR region of anti-human TNF-alpha antibody
<400> 8
gac gtc cag ttg acc cag tct cca tct gcc atg gct gca tct gta gga 48
Asp Val Gln Leu Thr Gln Ser Pro Ser Ala Met Ala Ala Ser Val Gly
1               5                   10                  15
gac aga gtc acc atc act tgt acg gcg agt tcg agc gtt agc ttc agt 96
Asp Arg Val Thr Ile Thr Cys Thr Ala Ser Ser Ser Val Ser Phe Ser

            20                  25                  30
tat tta cac tgg tat cag cag aaa cca ggg aaa gtc cct aag ctc tgg 144
Tyr Leu His Trp Tyr Gln Gln Lys Pro Gly Lys Val Pro Lys Leu Trp
        35                  40                  45
atc tat tct aca tcc aat ttg gca agt ggg gtc cca tcg agg ttc agc 192
Ile Tyr Ser Thr Ser Asn Leu Ala Ser Gly Val Pro Ser Arg Phe Ser
    50                  55                  60
ggc agt gga tct ggg aca gaa tac act ctc aca atc agc agc ctg cag 240
Gly Ser Gly Ser Gly Thr Glu Tyr Thr Leu Thr Ile Ser Ser Leu Gln
65                  70                  75                  80
cct gaa gat ttt gca act tat tac tgt cac cag tat ctt cgt tcc ccg 288
Pro Glu Asp Phe Ala Thr Tyr Tyr Cys His Gln Tyr Leu Arg Ser Pro
                85                  90                  95
tac act ttc ggc gga ggg acc aag gtg gag atc aaa 324
Tyr Thr Phe Gly Gly Gly Thr Lys Val Glu Ile Lys
            100                 105

<210> SEQ ID NO:9
<211> 30
<212> DNA
<213> Artificial Sequence
<220>
<223> Primer L1
<400> 9
aggtgtgacg tccagttgac ccagtctcca 30

<210> SEQ ID NO:10
<211> 54

<212> DNA

<213> Artificial Sequence

<220>

<223> Primer L2

<400> 10

ctgataccag tgtaaataac tgaagctaac gctcgaactc gccgtacaag tgat 54

<210> SEQ ID NO:11

<211> 42

<212> DNA

<213> Artificial Sequence

<220>

<223> Primer L3

<400> 11

gaccccactt gccaaattgg atgtagaata gatcaggagc tt 42

<210> SEQ ID NO:12

<211> 30

<212> DNA

<213> Artificial Sequence

<220>

<223> Primer L4

<400> 12

tgtgagagtg tattctgtcc cagatccact 30

<210> SEQ ID NO:13

<211> 39

<212> DNA

<213> Artificial Sequence
<220>
<223> Primer L5
<400> 13
gccgaaagtg tacggggaac gaagatactg gtgacagta 39

<210> SEQ ID NO:14
<211>
<212> DNA
<213> Artificial Sequence
<220> 20
<223> Primer L6
<400> 14
ctcatcagat ggcgggaaga 20

<210> SEQ ID NO:15
<211> 30
<212> DNA
<213> Artificial Sequence
<220>
<223> Primer H1
<400> 15
cagaattcac catggagttt gggctgagct 30

<210> SEQ ID NO:16
<211> 39
<212> DNA
<213> Artificial Sequence

<220>
<223> Primer H2
<400> 16
gacccagttc ataccatagt tagtgaaggt gtatccaga 39

<210> SEQ ID NO:17
<211> 48
<212> DNA
<213> Artificial Sequence
<220>
<223> Primer H3
<400> 17
gagtgggtgg catggataaa cacttataca ggtgagccaa cctacgca 48

<210> SEQ ID NO:18
<211> 48
<212> DNA
<213> Artificial Sequence
<220>
<223> Primer H4
<400> 18
gtctaaggaa atggtgaatc ggcccttgaa gtcgtctgcg taggttgg 48

<210> SEQ ID NO:19
<211> 48
<212> DNA
<213> Artificial Sequence
<220>

<223> Primer H5

<400> 19

tccctggccc cagtagtcaa atccgtcata atcatatctt gcacagta 48

## Description

### TECHNICAL FIELD

This invention relates to novel amino acid sequences participating in the binding of neutralizing antibody against a human TNFα to the antigen, genes encoding the same, gene recombinant antibodies containing these sequences, in particular, humanized antibodies, a method for producing these antibodies and pharmaceutical compositions containing these antibodies.

### PRIOR ART

Tumor necrosis factor α (TNFα) is an inflammatory cytokine exhibiting pleiotropic biological activity on cells (Proc. Natl. Acad. Sci. USA 72, 3666, 1975). TNFα, which is produced by many types of cells such as macrophages, mast cells and lymphoid cells, binds to a specific receptor occurring in the cell surface layer and thereby exhibits its effects (Annu. Rev. Biochem. 57, 505, 1988). Although TNFα exerts advantageous effects of, for example, killing tumor cells or virus-infected cells, some of its effects are obviously harmful to living organisms. For example, it is known that TNFα is the major factor causing septic shock (Science 234, 470, 1986). Moreover, systemic or topical overproduction of TNFα is implicated in diseases such as rheumatoid arthritis, multiple sclerosis and malaria (Proc. Natl. Acad, Sci. USA 89, 9784, 1992; J. Infect. Dis. 161, 1148, 1990; J. Exp. Med. 170, 607, 1989). It is expected that inhibition of the overproduction or the biological activity of TNFα will be useful in ameliorating the pathological conditions associated with these diseases.

It is known that an antibody generally has high affinity and high specificity for a given antigen. Neutralizing antibodies, which inhibit biological activity of antigens, are expected to be especially useful as drugs. There have been produced rabbit antiserum-derived polyclonal antibodies, and monoclonal antibodies of mouse, rat, etcs., which are employed for various purposes. However, these nonhuman-derived antibodies show high immunogenicity in human bodies. Accordingly, there arises a problem that human antibodies against these nonhuman-derived antibodies, which are produced as the result of administration of these antibodies to human bodies, not only interfere with the desired effects but also produce serious side effects due to immune reactions in patients. Thus, administration of these antibodies to patients is severely restricted.

An antibody molecule consists of two types of polypeptides. The polypeptide having the larger molecular weight is called the H chain, while the other polypeptide having the smaller molecular weight is called the L chain. Each of these polypeptides consists of a variable region forming an antigen-binding site and a constant region having almost the same structure within the same class of antibody. Furthermore, the variable region consists of complementarity determining regions (CDRs) closely relating to the formation of the antigen-binding site and regions called frameworks which are located among these CDRs. The H chain and the L chain have each 3 CDRs (i.e., 6 CDRs in total) which are respectively named CDR-1, CDR-2 and CDR-3 from the N-terminal end. It is known that the affinity and specificity of an antibody for an antigen are determined mainly by the amino acid sequences of these CDRs.

Recently, construction of human/mouse chimeric antibodies or humanized antibodies has been reported as a novel method for using antibodies as drugs (Nature 328, 323, 1988). A human/mouse chimeric antibody is a chimeric antibody having a mouse monoclonal antibody-derived variable region containing an antigen-binding site and an appropriate human antibody-derived constant region. Having the complete variable region of the original mouse antibody, it is expected that such a chimeric antibody will bind to its antigen with the same affinity and specificity as the original mouse antibody. Since this chimeric antibody has mouse-derived amino acid sequences exclusively in the variable region, it is also expected to have a lower immunogenicity compared to the original mouse antibody. On the other hand, a humanized antibody is prepared by transplanting CDRs of a mouse antibody into the variable region of a human antibody (Immunol. Today, 14, 243, 1993; Int. Rev. Immunol. 10, 241, 1993). In such a humanized antibody, the amino acid sequences of nonhuman origin are restricted to the CDRs. It is therefore considered that a humanized antibody prepared by transplanting mouse CDRs will have an even lower immunogenicity compared to chimeric antibodies.

A number of neutralizing antibodies against TNFα have been reported hitherto. Examples of these antibodies include a TNFα antibody which is efficacious in mouse rheumatoid arthritis models (Proc. Natl. Acad. Sci. USA 89, 9784, 1992), a TNFα antibody which ameliorates the pathological condition in mouse septic model (Nature 330, 662, 1987) and a TNFα antibody which is actually efficacious in human patients with rheumatoid arthritis (Lancet 344, 1105, 1994). However, these antibodies are either mouse-derived monoclonal antibodies or human/mouse chimeric antibodies. The amino acid sequences of these antibodies and the genes encoding them, and in particular the amino acid sequences of the CDRs participating in the recognition of TMFα (i.e., the antigen) and the genes encoding them, have not been known.

### SUMMARY OF THE INVENTION

One object of the present invention is to provide humanized antibodies against human TNFα and a method for producing them. Another object of the present invention is to provide pharmaceutical compositions which comprise these antibodies together with pharmaceutically acceptable carriers.

The present inventors developed a mouse monoclonal antibody MAB-3B10 specifically recognizing active human TNFα and disclosed it in JP(Kokai) SHO-63-253099. As the results of subsequent studies, they have determined the amino acid sequences of the H chain variable region and L chain variable region of MAB-3B10. Based on the determined amino acid sequences, recombinant antibodies against human TNFα and a method for producing the same are provided. The recombinant antibodies according to the present invention are preferably humanized antibodies.

### BRIEF DESCRIPTION OF THE DRAWINGS

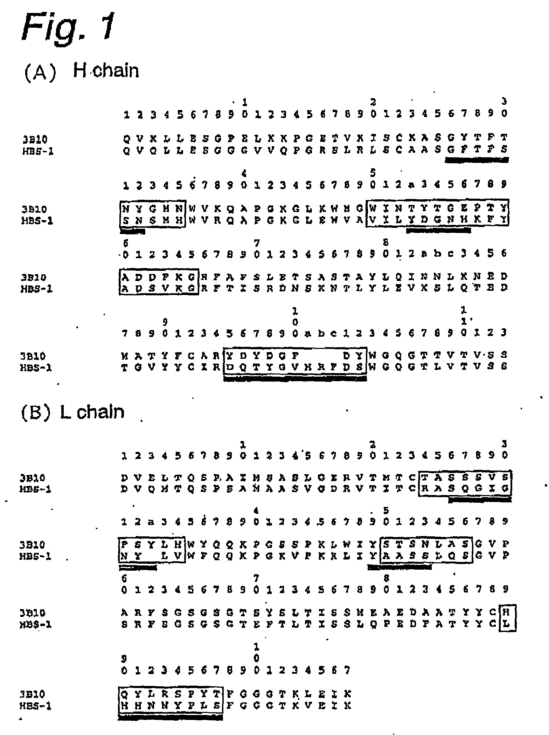

Fig. 1 is a diagram showing the amino acid sequences of the H chain and L chain variable regions of anti-human TNFα mouse neutralizing antibody 3B10. Amino acids are indicated by the one-letter notation method and numbered in accordance with the method of Kabat et al. (US Dept. Health and Human Services, US Government Printing Offices, 1991). The underlined parts indicate the CDRs determined in accordance with the method of Kabat et al. (US Dept. Health and Human Services, US Government Printing Offices. 1991), while the bordered parts indicate the CDRs determined in accordance with the method of Chothia et al. (J. Mol. Biol. 196, 901, 1987). In the present invention, amino acids belonging to either of these CDRs are regarded as "CDR". For reference, the amino acid sequences of the H chain and L chain variable regions of HBS-1 antibody corresponding to the sequences of 3B10 are also provided.

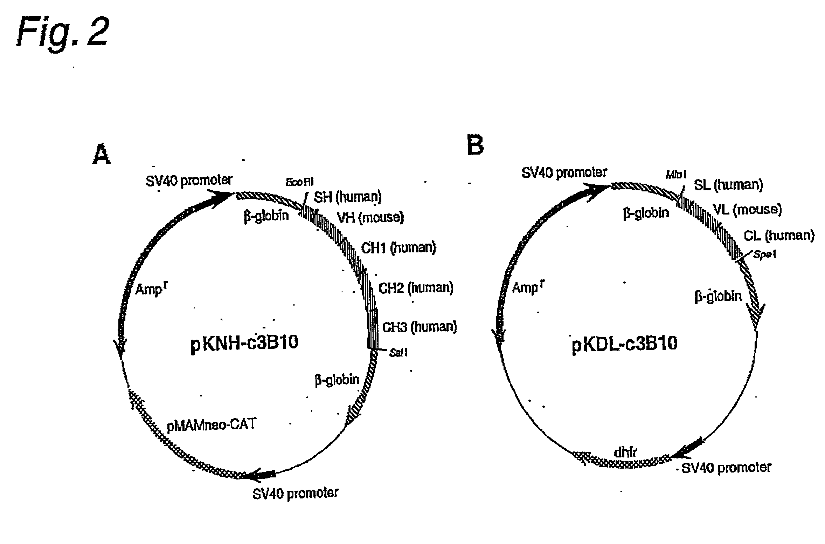

Fig. 2 provides model views of expression vectors for a human/mouse chimeric antibody against human TNFα. The vector A is an expression vector for the H chain of the human/mouse chimeric antibody against human TNFα, while the vector B is an expression vector for the L chain of the human/mouse chimeric antibody against human TNFα. In this figure, VH stands for the variable region of the H chain; SH stands for the signal region of the H chain, CH1, CH2 and CH3 stand respectively for 3 constant regions of the.H chain; VL stands for the variable region of the L chain; SL stands for the signal region of the L chain; and CL stands for the constant region of the L chain.

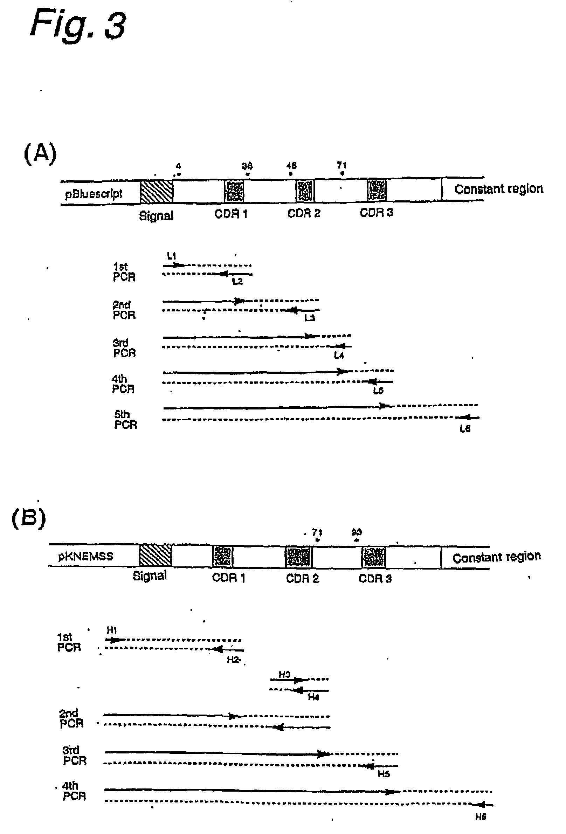

Fig. 3 provides diagrams showing procedures for constructing humanized anti-human TNFα antibodies. In this figure, the positions marked with figures and asterisks represent the positions wherein amino acids in the h3B10-1 frameworks have been substituted by amino acid residues of mouse 3B10. L1 to L6 stand respectively for PCR primers employed in constructing the L chain of h3B10-1, while H1 to H6 stand respectively for PCR primers employed in constructing the H chain of h3B10-1. This method is performed in accordance with an already reported method (Cancer Res. 53. 851, 1993).

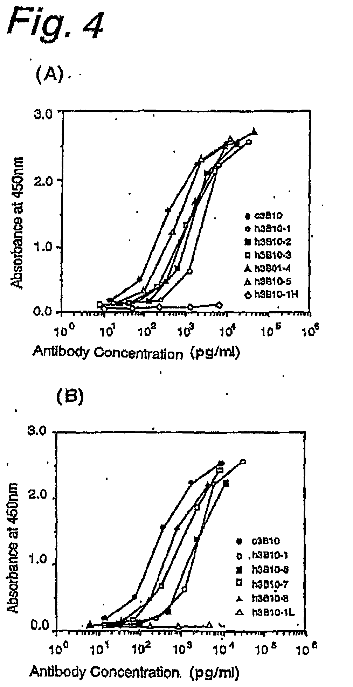

Fig. 4 provides graphs showing the affinities of humanized anti-human TNFα antibodies for human TNFα. specifically, these graphs show the affinities of the culture supernatants of COS-1 cells, into which genes encoding respective humanized anti-human TNFα antibodies have been transferred, 48 hours after the gene transfer. First, IgG having human IgG Fc is quantified by the FCA method. Then, the affinity of each humanized anti-human TNFα antibody for human TNFα is examined at various IgG concentrations by the ELISA method. The data are expressed in terms of absorbance at 450 nm.

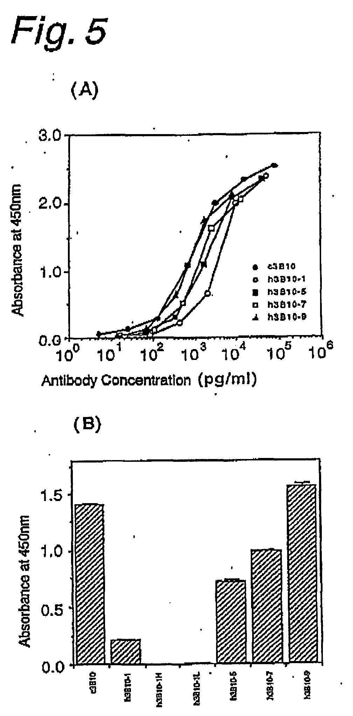

Fig. 5 provides graphs showing the affinities of humanized anti-human TNFα antibodies for human TNFα. Specifically, these graphs show the affinities of the culture supernatants of COS-1 cells, into which genes encoding respective humanized anti-human TNFα antibodies have been transferred, 48 hours after the gene transfer. First, IgG having human IgG Fc is quantified by the FCA method. Then, the affinity of each humanized anti-human TNFα antibody for human TNFα is examined at various IgG concentrations (A) or at 1.0 ng/ml (B) by the ELISA method. The data are expressed in terms of absorbance at 450 nm.
In (B), the data are expressed in terms of mean ± standard deviation.

### DETAILED DESCRIPTION OF THE INVENTION

The recombinant antibodies against human TNFα according to the present invention have at least one (preferably all) of CDR-1, CDR-2 and CDR-3 of the H chain variable region and the L chain variable region comprising the following amino acid sequences.
CDR-H1 of H chain: 
Gly-Tyr-Thr-Phe-Thr-Asn-Tyr-Gly-Met-Asn; (SEQ ID
NO:1);
CDR-H2 of H chain: Trp-Ile-Asn-Thr-Tyr-Thr-Gly-Glu-Pro-Thr-Tyr-Ala- Asp-Asp-Phe-Lys-Gly (SEQ ID NO:2);
CDR-H3 of H chain: Tyr-Asp-Tyr-Asp-Gly-Phe-Asp-Tyr (SEQ ID NO:3);
CDR-L1 of L chain: 
Thr-Ala-Ser-Ser-Ser-Val-Ser-Phe-Ser-Tyr-Leu-His
(SEQ ID NO:4);
CDR-L2 of L chain: Tyr-Ser-Thr-Ser-Asn-Leu-Ala-Ser (SEQ ID NO:5); and
CDR-L3 of L chain:

His-Gln-Tyr-Leu-Arg-Ser-Pro-Tyr-Thr (SEQ ID NO:6).

According to one embodiment, the present invention provides an antibody in which the H chain variable region contains the amino acid sequence consisting of the amino acids from the 1- to 113-positions in the amino acid sequence of Fig. 1(A) 3B10 (SEQ ID NO:7) or an amino acid sequence having substantially the same function as the above-described amino acid sequence, and the L chain variable region contains the amino acid sequence consisting of the amino acids from the 1- to 107-positions in the amino acid sequence of Fig. 1(B) 3B10 (SEQ ID NO:8) or an amino acid sequence having substantially the same function as the above-described amino acid sequence, and which recognizes human TNFα. (These sequences have been identified in the present invention as the sequences of the H chain and L chain variable regions of MAB-3B10).

The term "antibody" as used herein includes in its scope not only antibodies in the form usually occurring *in vivo* but also molecules having at least one antigen-binding site comprising the H chain or L chain variable region or a combination thereof. For example, proteins consisting of an H chain fragment and L chain pair such as Fab obtained by cleaving an antibody in the form usually occurring *in vivo* with papain, proteins consisting of two H chain fragment and L chain pairs such as F(ab')₂ similarly obtained by cleaving pepsin, and single-stranded antibodies consisting of an H chain fragment and an L chain bonded in series on a single peptide such as ScFv are all included in the scope. These "antibodies" other than those in the form usually occurring *in vivo* are sometimes obtained by cleaving antibodies in the form occurring *in vivo* with proteases. Alternatively, these antibodies may be constructed by using gene recombination techniques.

The present invention further includes in its scope fragments of the above-described antibody molecules according to the present invention which contain at least one of CDR-H1, CDR-H2, CDR-H3, CDR-L1, CDR-L2 and CDR-L3. For example, a peptide containing the H chain variable region of the amino acid sequence consisting of the amino acids from the 1- to 113-positions in Fig. 1(A) 3B10 or another amino acid sequence having substantially the same function is included in the scope of the present invention. Also, a peptide containing the L chain variable region of the amino acid sequence consisting of the amino acids from the 1- to 107-positions in Fig. 1(B) 3B10 or another amino acid sequence having substantially the same function is included in the scope of the present invention. Various artificial constructs which mimic antibodies can be produced by using one of these peptides or a combination thereof.

The expression "substantially the same function" as used herein means that the amino acid sequence of the complementarity determining region on an antibody molecule or its affinity for an antigen is substantially the same. In some cases, the affinity may be greater. Specifically, it is known that an antibody having substantially the same function can be obtained by substituting one to several amino acids in the framework of the variable region or in the constant region. It is also known that a humanized antibody sometimes has an increased affinity for its antigen. Accordingly, some antibody "derivatives" having "substantially the same function" can be constructed by, for example, retaining the amino acid sequences in the CDRs while substituting several amino acids in the framework of the variable region or in the constant region with other amino acids. It is widely known to substitute amino acids with other amino acids having similar characteristics for this purpose. For example, basic amino acids, acidic amino acids or aromatic amino acids may be substituted respectively by basic, acidic or aromatic amino acids.

It is further anticipated that some antibody "derivatives" having "substantially the same function" can be constructed by deleting or adding one to several amino acids in the framework of the variable region or in the constant region. These derivatives having substantially the same function also fall within the scope of the present invention.

The antibodies according to the present invention or fragments thereof can be produced by using gene recombination techniques.

Although the antibody according to the present invention may have any antibody other than the anti-human TNFα monoclonal antibody MAB-3B10 as the fundamental structure outside of the complementarity determining regions, it preferably has a human antibody as the fundamental structure. A case of producing an antibody according to the present invention using a human antibody as the fundamental structure will now be illustrated by way of example. First, an appropriate human monoclonal antibody is prepared. Next, a chimeric antibody is constructed by substituting the H chain and L chain variable regions of this antibody respectively with the H chain and L chain variable regions of the above-described anti-human TNFα monoclonal antibody (MAB-3B10). The human monoclonal antibody usable herein is not particularly restricted. For example, the human anti-HBs antibody known as HBS-1 (Gastroenterol Jpn. 19, 344, 1984; J. Immunol. Methods 222, 83, 1999) may be used. A method for producing chimeric antibodies is described in detail in Proc. Natl. Acad. Sci. USA, 81, 6951, 1984.

The chimeric antibody is then converted into a humanized antibody. In the H chain and L chain variable regions of the chimeric antibody, the framework regions outside the complementarity determining regions are respectively converted into the frameworks of a human antibody. That is to say, the method of Sato et al. (Cancer Res. 53. 851, 1993) is employed using DNAs encoding the L chain and H chain variable regions of HBS-1 as templates, as will be described in Example 3. Thus, DNAs encoding the L chain and H chain variable regions of MAB-3B10, the framework regions of which have been humanized, are amplified by using appropriate primers produced on the basis of the sequences of CDR-L1 to CDR-L3 and CDR-H1 to CDR-H3 of MAB-3B10 (for example, primers L1 to L6 of SEQ ID NOS:9 to 14, and primers H1 to H5 of SEQ ID NOS:15 to 19). By using the thus amplified DNAs respectively encoding the L chain and H chain variable regions, the variable regions of DNAs encoding the L chain and H chain of the above-described chimeric antibody are substituted again using the technique for constructing a chimeric antibody.

The obtained DNAs encoding the L chain and H chain of the humanized antibody are integrated into expression vectors and transformed into the same or separate host cells. The L chain and the H chain are thus separately or simultaneously expressed. The humanized antibody can thereby be secreted (Proc. Natl. Acad. Sci. USA, 84, 241, 1987; Cancer Res., 47, 999, 1987).

The recombinant antibody can be produced by, for example, transferring a gene encoding the recombinant antibody into COS-1 cells (SV40-transformed cells derived from African green monkey kidney) or CHO cells (Chinese hamster ovary-derived cells). Various vectors may be used to transfer the gene. For example, there may be used the eucaryotic cell expression vector pdKCR-dhfr (Biochem. Biophys. Res. Commun. 164, 39, 1989), modified as described in the following Examples. For expression of the DNA encoding the L chain or the H chain in the host cells, the vector may contain a promoter, a terminator and other factors. For secretion of the L chain and/or the H chain from the host, the DNA encoding the L chain and/or the H chain may be located downstream from a gene encoding a signal peptide compatible with the host cells.

For example, COS-1 cells are cultured in a medium such as 10% fetal calf serum (PCS)-containing Dulbecco's modified Eagle's medium (DMEM) in the presence of 5% CO₂ at 37°C. The method for gene transfer into the COS-1 cells and the method for culturing the cells after the gene transfer are described in, for example. Molecular Cloning, A Laboratory Manual (Second Edition, Cold Spring Harbor Laboratory Press, 1989).

To produce an antibody usable in medicines, it is desirable to culture the cells in serum-free medium to thereby avoid contamination with serum-derived bovine antibodies, etc. The anti-TNFα antibody thus secreted into the culture supernatant can be easily purified by a method generally employed in the art for purifying antibodies, for example, by using a resin binding protein A (Antibodies, A Laboratory Manual, Cold Spring Harbor Laboratory Press, 1988). As the host cells for industrial production, it is possible to use CHO cells, mouse myeloma cells Sp2/0. etc. With CHO cells, for example, a clone exhibiting a high productivity can be selected using a chemical such as MTX (Immunol, Lett. 64, 139, 1998). Such a strain exhibiting stable high productivity, if available, is useful in producing the recombinant anti-human TNFα antibody on an industrial scale.

The affinity of the antibody according to the present invention for human TNFα can be enhanced in some cases by substituting some of the amino acids in the framework regions of the variable regions with other amino acids. As will be shown in Example 4, such substitution can be performed by PCR using primers with appropriate variations thereinto.

The antibody molecules thus produced may be directly employed for use as antibodies in medicines. Alternatively, fragments containing the antigen-binding site, which are obtained by treatment with various proteases, may be employed. As described above, the antibodies may be used either in the form usually occurring *in vivo* or as fragments containing at least an antigen-binding site comprising the H chain or L chain variable regions of the antibody or a combination thereof. These antibodies may be produced by a gene recombination method. It is also possible to produce the antibodies by a gene recombination method followed by limited digestion with proteases. The production method is not particularly restricted.

The present invention further provides pharmaceutical compositions containing the above-described antibodies according to the present invention together with pharmaceutically acceptable carriers.

The pharmaceutical compositions according to the present invention may contain the antibodies according to the present invention together with substances which are used to maintain the activity of the antibodies for administration to human bodies, such as carriers and stabilizers composed of pharmaceutlcally acceptable ingredients. Examples of such carriers and stabilizers include human serum albumin and gelatin. The term "pharmaceutically acceptable" means causing no undesirable side effects such as nausea, vertigo or vomiturition and inducing no immune response to preparations even with frequent administration. The pharmaceutical compositions may be in the form of solutions prepared by dissolving the active ingredient in pharmaceutically acceptable appropriate solvents or diluents together with stabilizers. To control the concentration *in vivo,* the pharmaceutical compositions may further contain sustained-release ingredients such as microspheres or liposomes.

To prevent or treat diseases related to TNFα or overproduction thereof, the pharmaceutical compositions according to the present invention can be administered to patients suffering from, for example, septic shock, rheumatoid arthritis, multiple sclerosis or malaria. An appropriate administration route may be selected depending on the purpose from among systemic administration routes (intravenous administration, oral administration, intraperitoneal administration, subcutaneous administration, nasal administration, percutaneous administration, etc.) and topical administration routes (as ointments, lotions, etc.). The administration dose may be appropriately determined by the physician depending on the symptoms, age of the patient, etc.

### Examples

The present invention will now be described in greater detail by way of the following Examples. However, it is to be understood that the invention is not to be construed as restricted thereto.

### Example 1: Cloning of cDNAs of H chain and L chain variable regions of anti-human TNFα mouse neutralizing antibody 3B10

The total RNA of 3B10 cells (J. Immunol. Methods 96, 57, 1987) secreting a mouse monoclonal antibody against human TNFα was separated using an RNAzol B reagent (manufactured by BIOTBX Laboratories). Using this total RNA, cDNAs were synthesized with the use of a random hexamer and a reverse transcriptase (Super Script Preamplification System, manufactured by GIBCO BRL). From among the cDNAs thus obtained, cDNAs encoding the H chain and L chain variable regions were amplified by the polymerase chain reaction (PCR) method. The cDNA of the L chain variable region was amplified using amplification primers synthesized according to the sequences reported by Huse et al. (Science 246, 1275, 1989), while the cDNA of the H chain variable region was amplified with the combined use of a 5'-primer (5'-AGGTGAAGCTNGTGGAG/ATCTGG-3') designed based on the H chain amino acid sequence reported by Kabat et al. (US Dept. Health and Human Services, US Government Printing Offices, 1991) and a 3'-primer of Huse et al. A thermostable DNA polymerase (AmpliTaq DNA polymerase, manufactured by Perkin-Elmer) and a thermal cycler (TRIO-Thermo-block, manufactured by Biometra) were used for the PCR. The nucleotide sequences of the cDNAs thus obtained were analyzed in accordance with the method of Sanger et al. (Proc. Natl. Acad. Sci. USA 74, 5463, 1977). Fig. 1 shows the nucleotide sequences of the H chain and L chain variable regions of the 3B10 antibody thus obtained. The CDRs were then identified in accordance with the method of Kabat et al. (US Dept. Health and Human Services, US Government Printing Offices, 1991) or the method of Chothia et al. (J. Mol. Biol. 196, 901, 1987). In the present invention, an amino acid sequence contained in any of the CDRs is referred to as a "CDR".

### Example 2: Construction of expression vector of human/mouse chimeric antibody against anti-human TNFα

Multicloning sites (Eco RI, Mlu I, Spe I and Sal I) were transferred into an expression vector for eucaryotic cells pdKCR-dhfr (Biochem. Biophys. Res. Commun. 164, 39, 1989) to give a vector which will hereinafter be referred to as pKDEMSS vector. Next, the dihydrofolate reductase (DHFR) region of pKDEMSS was substituted with the neomycin resistance (neor) region of pMAMneoCAT (manufactured by CLONTECH) to give a vector which will hereinafter be referred to as pKNEMSS vector. A chimeric H chain expression vector was constructed by integrating the γ1 chain signal sequence of the human anti-HBs antibody HBS-1 (Gastroenterol. Jpn. 19, 344, 1984; J. Immunol. Methods 222, 83, 1999), the H chain variable region of 3B10 obtained in Example 1 and the γ1 chain constant region of HBS-1 into the pKNEMSS vector in that order from the N terminal end. The vector thus obtained will hereinafter be referred to as PKNH-c3B10. Similarly, a chimeric L chain expression vector was constructed by integrating the κ chain signal sequence of HBS-1, the L chain variable region of 3B10 obtained in Example 1 and the κ chain constant region of HBS-1 into pKDEMSS vector in that order from the N terminal end. The vector thus obtained will hereinafter be referred to as pKDL-c3B10. Fig. 2 shows the structures of pKNH-c3B10 and pKDL-c3B10.

### Example 3: Construction of humanized anti-human TNFα antibody

In accordance with the method of Sato et al. (Cancer Res. 53, 851, 1993), an antibody gene was constructed by substitution of six CDRs of the mouse antibody 3B10 into the corresponding positions of human IgG. As Fig. 3 shows, a cDNA fragment of the L chain variable region was constructed by performing PCR 5 times using the cDNA of the L chain of the HBS-1 antibody as a template. In the first PCR, amplification was carried out using the primers L1 and L2. The second PCR was carried out in combination with the primer L3, using the amplified fragment as the 5' primer. Three PCR were carried out using up to the primer L6, to obtain the target L chain variable region cDNA fragment as the final product. Also, an H chain variable region cDNA fragment was constructed by repeating PCR with the use of the H chain cDNA of HBS-1 as a template. In this case, however, the 3' primer employed in the second PCR was prepared by performing PCR using 2 types of primers overlapping each other. The variable regions of the anti-human TNFα human/mouse chimeric antibody expression vectors pKDL-c3B10 and pKNH-c3B10 obtained in Example 2 were then substituted with these L chain and H chain variable region cDNA fragments as the final amplification products, thereby giving humanized antibody expression vectors. An anti-human TNFα humanized antibody can be secreted by culturing appropriate host cells transformed simultaneously by these vectors under the conditions as allow expression of the antibody (see Example 4). The humanized antibody thus obtained was named h3B10-1.

Table 1 shows the primers used in this Example. In these primers, the amino acids at the 4-, 36-, 48- and 71-positions of the L chain framework region and the amino acids at the 71- and 93-positions of the H chain have been substituted respectively by the corresponding amino acid residues of mouse 3B10.

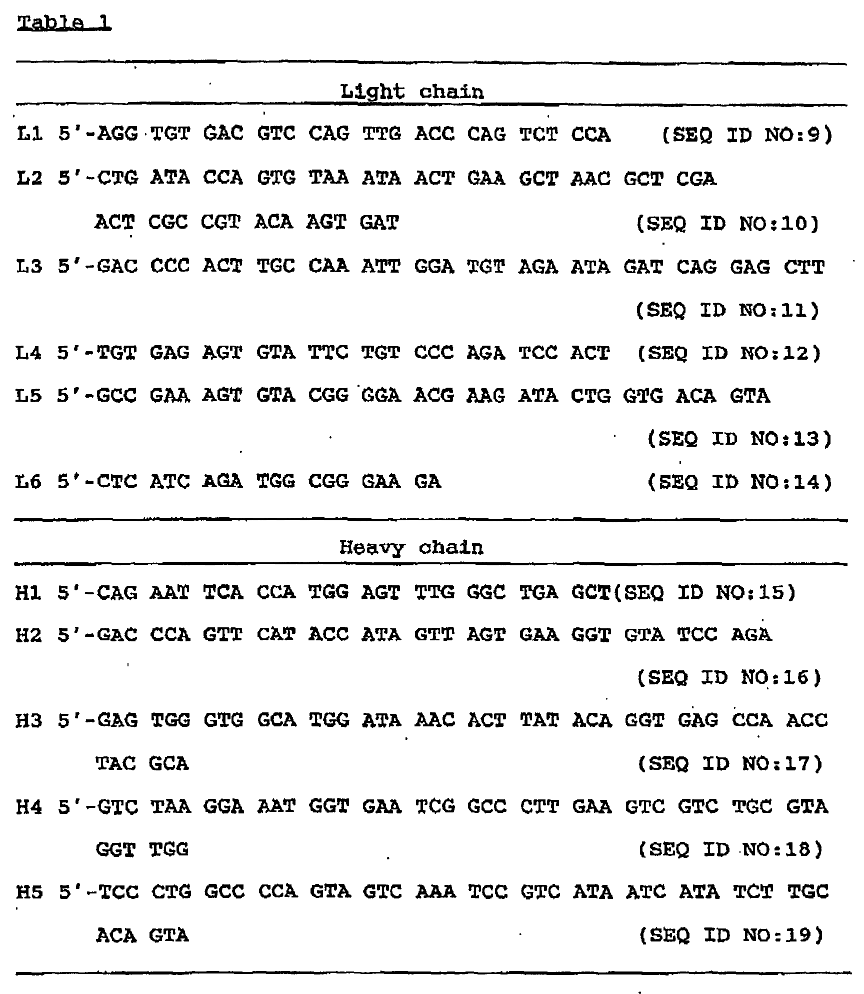

### Example 4: Construction of humanized anti-human TNFα antibody derivatives

Eight humanized 3B10 antibody derivatives were produced by further mutating h3B10-1. Mutations were introduced into the H chain and L chain framework regions. Specifically, h3B10-1 was employed as a template and PCR was carried out using primers carrying introduced mutations. A thermostable DNA polymerase (AmpliTaq DNA polymerase, manufactured by Perkin-Elmer) and a thermal cycler (TRIO-Thermo-block, manufactured by Biometra) were used in the PCR. Table 2 summarizes the differing points of the amino acid sequences of the produced 8 humanized antibodies (named h3B10-2 to 9), h3B10-1H and h3B10-1L. The antibody having the same L chain as h3B10-1 and the actual H chain framework regions of HBS-1 was named h3B10-1H, while the antibody having the same H chain as h3B10-1 and the actual L chain framework regions of HBS-1 was named h3B10-1L.

**Table 2:**

| Structures of humanized anti-TNFα antibody framework regions | | | | | | | | | | | |
|---|---|---|---|---|---|---|---|---|---|---|---|
| | Amino acid residues of H chain | | | | | Amino acid residues of L chain | | | | | |
| Antibody | 3 | 46 | 71 | 78 | 93 | 3 | 4 | 36 | 46 | 47 | 71 |
| m3B10 | K | K | L | A | A | E | L | Y | L | W | Y |
| c3B10 | K | K | L | A | A | E | L | Y | L | W | Y |
| HBS-1 | Q | E | R | L | I | Q | M | F | R | L | F |
| h3B10-1 | Q | E | L | L | A | Q | L | Y | L | L | Y |
| h3b10-1H | Q | E | R | L | I | Q | L | Y | L | L | Y |
| h3B10-1L | Q | E | L | L | A | Q | M | F | R | L | F |
| h3B10-2 | K | E | L | L | A | Q | L | Y | L | L | Y |
| h3B10-3 | Q | K | L | L | A | Q | L | Y | L | L | Y |
| h3B10-4 | Q | E | L | A | A | Q | L | Y | L | L | Y |
| h3B10-5 | K | K | L | A | A | Q | L | Y | L | L | Y |
| h3B10-6 | Q | E | L | L | A | E | L | Y | L | L | Y |
| h3B10-7 | Q | E | L | L | A | Q | L | Y | L | W | Y |
| h3B10-8 | Q | E | L | L | A | E | L | Y | L | W | Y |
| h3B10-9 | K | K | L | A | A | Q | L | Y | L | W | Y |

In Table 2, amino acids are indicated by the one-letter notation method and numbered in accordance with the method of Kabat et al. (US Dept. Health and Human Services, US Government Printing Offices, 1991). The underlined sections represent the mouse-derived sequences.
- m3B10:: original mouse anti-TNFα antibody:
- c3B10:: human/mouse chimeric anti-TNFα antibody;
- HBS-1:: human antibody against HBs;
- h3B10-1H:: antibody having the same L chain as h3B10-1 and H chain frameworks identical with HBS-1;
- h3B10-1L:: antibody having the same H chain as h3B10-1 and L chain frameworks identical with HBS-1.

### Example 5: Expression of humanized anti-human TNFα antibody and its derivatives and analysis thereof

3.0 x 10⁵ COS-1 cells (obtained from ATCC) were inoculated into a 35 mm Petri dish and precultured for 18 hours. Two µg portions of the pairs of respective H chain expression vectors and L chain expression vectors corresponding to the humanized anti-human TNFα antibody and the human/mouse chimeric antibodies constructed in Examples 2 to 4 (i.e., 9 types in total) were simultaneously transferred into the COS-1 cells using 10 µl of a Lipofectamine Reagent (manufactured by GIBCO BRL). The affinities for human TNFα of the recombinant antibodies secreted from the gene-introduced cells were examined by the ELISA method. Also, the human IgG in the culture supernatant was quantified by the fluorescence concentration analyzer (FCA) method. The ELISA method was carried out in the following manner. First, 60 wells at the center of a 96-well plate were filled with 10 µg/ml human TNF-α which was then immobilized by incubating at room temperature for 18 hours. After washing with a washing buffer (phosphate buffered saline (PBS) containing 0.1% Tween 20) three times, the wells were blocked with PBS containing 1% of BSA for 2 hours. After washing with PBS-T three times, the human TNF-α was reacted with each of the COS-1 cell culture supernatants for 2 hours. After washing in the same manner, the antibody binding with TMF-α was reacted with peroxidase-labeled goat anti-human IgG Fc antibody (manufactured by Jackson ImmunoResearch Laboratories) or peroxidase-labeled goat anti-mouse IgG Fc antibody (manufactured by Caltag Laboratories) and detected in accordance with the method proposed in the literature (J. Immunol. Methods 143, 89, 1991). Separately, the FCA method was carried out in accordance with an already reported method (Biochem. Biophys. Res. common. 193, 886, 1993) using FCA particles coated with goat anti-human IgG Fc antibody and FITC-labeled goat anti-human IgG Fc antibody. Quantification was performed by using human IgG of a known concentration as the standard. The affinity for human TNFα of each humanized antibody expressed in the COS-1 cells was represented as ELISA reaction doses at various IgG concentrations.

As a result, each humanized antibody exhibited strong binding activity to human TNFα, as shown in Figs. 4 and 5, with h3B10-9 exhibiting the strongest activity which was comparable to the human/mouse chimeric antibody. The antibody h3B10-1 also exhibited binding activity, though somewhat lower than the activity of the human/mouse chimeric antibody. Antibodies h3B10-1H and h3B10-1L prepared as controls show no affinity for human TNFα.

### Effect of the Invention

It has been demonstrated that humanized antibodies retaining binding activity to human TNFα can be obtained by substitution of the 6 CDR amino acid sequences and genes encoding the same, which are provided for the first time by the present invention, into the corresponding positions of human IgG. For use in medicines, the humanized anti-TNFα antibodies obtained by the present invention show extremely lower immunogenicity and improved safety compared with antibodies derived from animals such as mice. The present invention makes it possible to prepare a large amount of humanized antibodies which recognize human TNFα upon administration to patients suffering from various diseases associated with TNFα.

## Claims

1. An H chain polypeptide of a recombinant antibody against human TNFα, or its fragment, which has at least one of the following amino acid sequences:
a) the amino acid sequence represented by SEQ ID NO:1 as CDR-H1,
b) the amino acid sequence represented by SEQ ID NO:2 as CDR-H2; and
c) the amino acid sequence represented by SEQ ID NO:3 as CDR-H3.

2. An H chain polypeptide of a recombinant antibody against human TNFα which contains the H chain variable region of an antibody against human TNFα comprising the amino acid sequence represented by SEQ ID NO:7 or an amino acid sequence derived from said amino acid sequence by deletion, addition or substitution of one to several amino acids in a region other than the amino acid sequences represented by SEQ ID NOS:1 to 3, or its fragment.

3. An L chain polypeptide of a recombinant antibody against human TNFα which has at least one of the following amino acid sequences:
a) the amino acid sequence represented by SEQ ID NO:4 as CDR-L1;
b) the amino acid sequence represented by SEQ ID NO:5 as CDR-L2; and
c) the amino acid sequence represented by SEQ ID NO:6 as CDR-L3.

4. An L chain polypeptide of a recombinant antibody against human TNFα which contains the L chain variable region of an antibody against human TNFα comprising the amino acid sequence represented by SEQ ID NO:8 or an amino acid sequence derived from said amino acid sequence by deletion, addition or substitution of one to several amino acids in a region other than the amino acid sequences represented by SEQ ID NOS:4 to 6.

5. A gene encoding an H chain polypeptide or its fragment as claimed in claim 1 or 2.

6. A gene encoding an L chain polypeptide as claimed in claim 3 or 4.

7. An expression vector having the gene(s) as claimed in claim 5 and/or claim 6 incorporated thereinto.

8. A method for producing a recombinant antibody against human TNFα which comprises transforming host cells by the expression vector as claimed in claim 7, culturing the host cells under such conditions as allow expression of the antibody against human TNFα, and collecting the antibody thus produced by the host cells.

9. A recombinant antibody against human TNFα which can be obtained by a gene recombination technique using the gene(s) as claimed in claim 5 and/or claim 6 or the method as claimed in claim 8, or its fragment.

10. A pharmaceutical composition comprising the antibody as claimed in claim 9 or its fragment together with a pharmaceutically acceptable carrier.
